# EUROPÄISCHE PATENTSCHRIFT

(11) **EP 2 874 601 B1**
(45) Veröffentlichungstag und Bekanntmachung des Hinweises auf die Patenterteilung: **04.05.2016**
(21) Anmeldenummer: 13747823.6
(22) Anmeldetag: 05.08.2013
(51) Int. Cl.: A61K 9/00, A61K 47/36, A61K 9/20, A61Q 11/00, A61K 31/728, A61K 31/731

(54) **ZUSAMMENSETZUNG, INSBESONDERE PHARMAZEUTISCHE ZUSAMMENSETZUNG, INSBESONDERE ZUR VERABREICHUNG BEI HEISERKEIT**
COMPOSITION, IN PARTICULAR A PHARMACEUTICAL COMPOSITION, IN PARTICULAR FOR ADMINISTRATION IN HOARSENESS
COMPOSITION, EN PARTICULIER COMPOSITION PHARMACEUTIQUE, DESTINÉE NOTAMMENT À ÊTRE ADMINISTRÉE EN CAS D'ENROUEMENT

(30) Priorität: 04.10.2012 DE 102012019413; 17.01.2013 DE 102013000699
(43) Veröffentlichungstag der Anmeldung: 27.05.2015
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE); GALÁN SOÚSA, José, 13465 Berlin (DE); UNKAUF, Markus, 50670 Köln (DE); PLOCH, Michael, 16565 Lehnitz (DE)
(74) Vertreter: Von Rohr Patentanwälte Partnerschaft mbB
(86) Internationale Anmeldenummer: PCT/EP2013/066363
(87) Internationale Veröffentlichungsnummer: WO 2014/053262

(56) Entgegenhaltungen:
- EP-A2- 2 251 016
- WO-A1-2008/009956
- CH-A5- 691 030
- US-A1- 2002 183 278
- US-A1- 2003 013 643
- ECCLES R. ET AL.: "Efficacy and safety of an antiviral iota-carrageenan nasal spray: a randomized, double-blind, placebo-controlled exploratory study in volunteers with early symptoms of the common cold", RESPIRATORY RESEARCH, Bd. 11, 108, 10. August 2010 (2010-08-10), XP002719199,
- GRASSAUER A. ET AL.: "Iota-carrageenan is a potent inhibitor of rhinovirus infection", VIROLOGY JOURNAL, Bd. 5, 107, 26. September 2008 (2008-09-26), XP002719200,

## Beschreibung

Die vorliegende Erfindung betrifft das Gebiet der Medizin, insbesondere der Behandlung von Heiserkeit bzw. entzündlichen Erkrankungen des Mund- und Rachenraums (wie z. B. Halsschmerzen).

Insbesondere betrifft die vorliegende Erfindung eine Zusammensetzung, vorzugsweise eine pharmazeutische Zusammensetzung, welche sich für die topische Behandlung von Heiserkeit bzw. entzündlichen Erkrankungen des Mund- und Rachenraums (wie z. B. Halsschmerzen) eignet. Darüber hinaus betrifft die vorliegende Erfindung die erfindungsgemäßen Verwendungen der Zusammensetzung nach der Erfindung.

Unter Heiserkeit bzw. Dysphonie wird üblicherweise eine Beeinträchtigung des stimmlichen Teils der Phonation (Artikulation) verstanden, wobei in diesem Zusammenhang im Allgemeinen eine Funktionsstörung des Kehlkopfes und des Ansatzrohres vorliegt. Die Stimme klingt bei Heiserkeit je nach Befund und Ausprägung heiser, rau, belegt oder behaucht und ist nicht mehr "flexibel", d. h. Klangfarbe, Tonhöhe und Lautstärke können vom Betroffenen nur im verminderten Umfang variiert werden. Insbesondere tritt bei Heiserkeit oftmals auch das Symptom der Mundtrockenheit (Xerostomie) auf.

Die der Heiserkeit zugrundeliegenden Ursachen sind vielfältig und können sowohl funktionell als auch organisch bedingt sein. Zu den funktionellen Ursachen gehören insbesondere eine allgemeine Überbeanspruchung der Stimme, beispielsweise durch Singen, oder stimmschädigende Sprechgewohnheiten. Zu den häufigsten organischen Ursachen gehören insbesondere entzündliche Erkrankungen des Mund- und Rachenraums, wie sie beispielsweise bei Erkältungen oder grippalen Infekten auftreten. Darüber hinaus kommen als organische Ursachen für Heiserkeit auch Lähmungen oder Gewebsneubildungen in Betracht. In seltenen Fällen ist es auch möglich, dass angeborene Fehlbildungen im Bereich des Kehlkopfes zu Heiserkeit führen.

Sowohl durch funktionelle als auch durch organische Ursachen, insbesondere entzündliche Erkrankungen des Mund- und Rachenraums, hervorgerufene Heiserkeit ist für den betroffenen Patienten oftmals mit einer unangenehmen Schmerz- und Entzündungssymptomatik verbunden. Auch können schmerzhafte Schluckbeschwerden auftreten, was insbesondere die Nahrungsaufnahme erschwert. Betroffene empfinden darüber hinaus die Beeinträchtigung der Phonation, welche charakteristisch für Heiserkeit ist, als äußerst störend.

Die Behandlung von Heiserkeit, sowohl bei funktionellen als auch bei organischen Ursachen, insbesondere entzündlichen Erkrankungen des Mund- und Rachenraums, erfolgt üblicherweise - in Abhängigkeit von der Schwere des Krankheitsbildes - durch topische Therapie.

Mit den im Stand der Technik bekannten Maßnahmen lässt sich jedoch oftmals kein zufriedenstellender Therapieerfolg erzielen, wie im Folgenden beschrieben.

Die Therapiemaßnahmen beschränken sich nämlich im Allgemeinen auf die Gabe von "Hausmitteln", wie beispielsweise heißer Milch mit Honig oder Gurgellösungen auf Basis von Kochsalz oder Salbei. Derartige Behandlungsmethoden gewährleisten jedoch oftmals keine zufriedenstellende Linderung der Beschwerden. Einige der altbekannten Hausmittel, wie insbesondere heiße Milch mit Honig, sind sogar abträglich, da sie eine verstärkte Schleimbildung im Hals bewirken und somit dem Heilungsprozess bzw. der Regeneration entgegenstehen.

Darüber hinaus kommen häufig handelsübliche Lutschtabletten zum Einsatz, welche zwar eine kurzzeitige Linderung verschaffen, deren Wirkung jedoch üblicherweise äußerst schnell nachlässt.

Insbesondere bieten sämtliche im Stand der Technik angewandten Behandlungskonzepte für Heiserkeit keine ausreichende Linderung der Schmerz- und Entzündungssymptomatik, vor allem wenn der Heiserkeit organische Ursachen, wie entzündliche Erkrankungen des Mund- und Rachenraums, zugrundeliegen.

Entsprechendes gilt auch für die Behandlung von Halsschmerzen oder anderen entzündlichen Erkrankungen des Mund- und Rachenraums. Auch hier sind die Therapeutika des Standes der Technik oftmals nicht effizient.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf. Solche entzündlichen Prozesse des Mund- und Rachenraums sind oftmals mit einer für den betroffenen Patienten unangenehmen Schmerzsymptomatik verbunden. Nicht beschränkende Beispiele für derartige entzündliche Erkrankungen des Hals-/Rachenraums sind Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Solche Erkrankungen des Hals-/Rachenraums sind oftmals von schmerzhaften Schluckbeschwerden und Halsschmerzen begleitet. Auch Entzündungen im Bereich der Mundhöhle, wie z. B. Stomatitis, Gingivitis, Mundschleimhautläsionen und dergleichen, sind oftmals von Schmerzen begleitet. Für weitere diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden. Der Begriff "entzündliche Erkrankungen des Mund- und Rachenraums" ist somit sehr weit zu verstehen und umfasst sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im allgemeinen durch topische und gegebenenfalls zusätzliche systemische Therapie in schwerwiegenderen Fällen. Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können. Diese Maßnahmen sind meist nicht ausreichend effizient und nachhaltig.

Die CH 691 030 A5 betrifft eine Zusammensetzung auf Basis eines wässrigen Gels, welche zur topischen Anwendung vorgesehen ist und eine Wirkstoffkombination auf Basis von Carrageen einerseits sowie Hyaluronsäure andererseits aufweist.

Die WO 2008/009956A1 betrifft eine Zusammensetzung für die orale Applikation, wobei die Zusammensetzungen einen pH-Wert im Bereich von 3 bis 8,5 und als pharmakologisch wirksame Substanzen Bioflavonoide und Fruchtsäuren, Natriumhyaluronat sowie darüber hinaus Wasser und einen pharmazeutisch verträglichen Exzipienten aufweisen.

Weiterhin betrifft die US 2002/0183278A1 Zusammensetzungen, welche als aktive Inhaltsstoffe Hyaluronsäure und Polyvinylpyrrolidon enthalten und zur Behandlung von entzündlichen bzw. schmerzhaften Zuständen der Schleimhäute geeignet sind.

Der vorliegenden Erfindung liegt daher die Aufgabe zugrunde, eine Zusammensetzung, insbesondere eine pharmazeutische Zusammensetzung, bereitzustellen, welche die zuvor geschilderten Nachteile des Standes der Technik zumindest weitgehend vermeidet oder aber wenigstens abschwächt.

Im Speziellen liegt der vorliegenden Erfindung die Aufgabe zugrunde, eine Zusammensetzung bereitzustellen, welche sich zur effizienten topischen Behandlung von Heiserkeit bzw. entzündlichen Erkrankungen des Mund- und Rachenraums (z. B. Halsschmerzen) eignet.

Darüber hinaus liegt der vorliegenden Erfindung die Aufgabe zugrunde, eine Zusammensetzung bereitzustellen, welche im Rahmen der Behandlung von Heiserkeit bzw. entzündlichen Erkrankungen des Mund- und Rachenraums (z. B. Halsschmerzen) eine hervorragende und lang andauernde Linderung der Schmerz- und Entzündungssymptomatik erlaubt und zudem dem Symptom der Mundtrockenheit entgegenwirkt.

Zur Lösung der zuvor geschilderten Aufgabe schlägt die vorliegende Erfindung eine Zusammensetzung nach Anspruch 1 vor; weitere vorteilhafte Ausgestaltungen sind Gegenstand der diesbezüglich abhängigen Ansprüche.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** Erfindungsaspekt - eine Zusammensetzung, insbesondere pharmazeutische Zusammensetzung, in Form einer festen Dosierung, welche zur topischen Behandlung von Heiserkeit und/oder entzündlichen Erkrankungen des Mund- und Rachenraums geeignet ist,
wobei die Zusammensetzung in Kombination und jeweils in wirksamen, insbesondere pharmazeutisch wirksamen Mengen
(a) mindestens ein virustatisches oder antiviral wirksames Polysaccharid ("Komponente (a)");
(b) Hyaluronsäure oder deren Salze ("Komponente (b)"); und
(c) mindestens einen Schaumbildner ("Komponente (c)"); aufweist.

Die Anmelderin hat nunmehr in überraschender Weise herausgefunden, dass eine Zusammensetzung, vorzugsweise eine pharmazeutische Zusammensetzung, welche eine Wirkstoffkombination auf Basis mindestens eines virustatischen oder antiviral wirksamen Polysaccharids mit Hyaluronsäure oder deren Salzen aufweist, eine hervorragende Linderung von Heiserkeit und anderen entzündlichen Erkrankungen des Mund- und Rachenraums sowie der jeweils damit einhergehenden Schmerz- und Entzündungssymptomatik verschafft und gleichzeitig Mundtrockenheit in effektiver Weise lindert. In diesem Zusammenhang hat sich insbesondere gezeigt, dass die Wirkung der Einzelwirkstoffe in der erfindungsgemäßen Kombination über die der Einzelwirkstoffe bei alleinigem Einsatz in signifikanter Weise hinausgeht, was als Indiz für das Vorliegen eines synergistischen Effekts zu werten ist.

Die Begriffe "pharmazeutische Zusammensetzung", "pharmazeutische Zubereitung" oder dergleichen, wie sie im Rahmen der vorliegenden Erfindung verwendet werden, sind sehr umfassend zu verstehen und bezeichnen nicht nur pharmazeutische Präparate bzw. Pharmazeutika, sondern auch sogenannte Medizinprodukte, homöopathische Mittel, Nahrungsergänzungsmittel oder dergleichen.

Im Rahmen der vorliegenden Erfindung werden unter dem Begriff "Polysaccharide" ("Komponente (a)") makromolekulare Kohlenhydrate, deren Moleküle aus einer Zahl von mindestens > 10 glykosidisch miteinander verknüpften Monosaccharid-Molekülen bestehen, zusammengefasst. Zu den Polysacchariden gehören zahlreiche Biopolymere bzw. Polymere biogenen Ursprungs, welche äußerst variable chemische bzw. pharmakologische Eigenschaften besitzen können. Polysaccharide dienen vorwiegend als Reservestoffe und liegen üblicherweise als Homopolymere bzw. Homoglykane vor, welche jeweils eine Art von Monosaccharid zugrundeliegt. Zudem existieren Polysaccharide, welche unter Gelbildung große Mengen Wasser binden können und demzufolge als sogenannte Schleimstoffe bezeichnet werden. Zu den Schleimstoffen gehörende Polysaccharide liegen meistens als Heteropolymere oder Heteroglykane auf Basis verschiedenartiger Monomereinheiten vor. Bekannte Schleimstoffe sind Pektine, Mannane, Galaktane, Xylane, Hyaluronsäure bzw. Glykosaminoglykane sowie zahlreiche Polymere auf Basis von sulfonierten Galaktanen, wie beispielsweise Carrageene, sowie Uronsäuren. Für die erfindungsgemäße Zusammensetzung hat sich der Einsatz von solchen gelbildenden Polymeren als besonders vorteilhaft erwiesen, da diese eine besonders starke Affinität zur Mundschleimhaut bzw. Rachenschleimhaut zeigen und sozusagen einen Schutzfilm bilden, welcher die Schleimhaut einerseits vor dem Eindringen von Krankheitserregern, wie beispielsweise Erkältungsviren, und andererseits einem Flüssigkeitsverlust bzw. Mundtrockenheit vorbeugt. Darüber hinaus existieren auch Polysaccharide, insbesondere Schleimstoffe, welche über antivirale Eigenschaften verfügen. Der Einsatz derartiger Polysaccharide ist für die erfindungsgemäße Zusammensetzung vorgesehen.

Für weitergehende Einzelheiten zum Begriff der "Polysaccharide" kann verwiesen werden auf Römpp, Chemielexikon, 10. Auflage, Georg Thieme Verlag, Stuttgart / New York, Seite 3503, Stichwort: "Polysaccharide", sowie auf die darin referierte Literatur, deren diesbezüglicher Inhalt hiermit vollumfänglich durch Bezugnahme eingeschlossen ist.

Bei den erfindungsgemäß verwendeten vorzugsweise sauren Glykosaminoglykanen ("Komponente (b)") handelt es sich um lineare Mucopolysaccharide, welche aus sich wiederholenden Sacchariden gebildet werden. Im Allgemeinen bestehen die Saccharid-Einheiten aus einer Uronsäure, wie insbesondere Glucuronsäure, aber auch Iduronsäure oder Uronsäure, welche 1,3-glykosidisch mit einem Aminozucker, wie N-Acetylglucosamin, verknüpft sind. Die Saccharid-Einheiten zur Ausbildung der Kette als solcher wiederum sind 1,4-glykosidisch miteinander verbunden. Insbesondere kann es auch vorgesehen sein, dass die Glykosaminoglykane mit Schwefelsäure oder Essigsäure weiter verestert sind. Die in Rede stehenden Glykosaminoglykane liegen häufig in Assoziation mit biologischen Makromolekülen vor und können beispielsweise in großer Zahl kovalent an Proteine gebunden vorkommen. Auf diese Weise können sie das Gerüst vieler faserbildender Stoffe, wie z. B. Fibrillin, im Körper ausbilden. Ein erfindungsrelevanter Vorteil bzw. eine erfindungsrelevante Eigenschaft der Glykosaminoglykane liegt insbesondere in ihrer Fähigkeit, in großen Mengen Wasser aufnehmen zu können, so dass bei topischer Applikation ein Austrocknen der Haut bzw. Schleimhaut verhindert wird.

Im Rahmen der vorliegenden Erfindung ist es vorgesehen, dass als Glykosaminoglykan Hyaluronsäure oder deren Salze eingesetzt wird. Hyaluronsäure stellt im menschlichen bzw. tierischen Körper einen wichtigen Bestandteil des Bindegewebes dar und spielt darüber hinaus eine Rolle bei der Zellproliferation und Zellmigration. Im Rahmen der vorliegenden Erfindung hat sich insbesondere die zellproliferative Wirkung in Bezug auf eine schnelle Regenerierung des Gewebes als vorteilhaft erwiesen. Weiterhin besitzt auch Hyaluronsäure die Fähigkeit, äußerst große Mengen an Wasser zu binden: So können mit 1 g Hyaluronsäure bis zu 6 Liter Wasser gebunden werden. Durch die Bindung großer Mengen an Wasser kann einer Austrocknung der Haut bzw. Schleimhaut in effizienter Weise vorgebeugt werden, so dass eine beschleunigte Regeneration des Gewebes ermöglicht wird und die Haut bzw. Schleimhaut darüber hinaus elastisch bleibt.

Chemisch gesehen handelt es sich bei Hyaluronsäure um eine makromolekulare Kette aus Disacchariden auf Basis von D-Glucuronsäure und N-Acetyl-D-Glucosamin, welche über eine beta-1,3-glykosidische Bindung miteinander verknüpft sind. Die Disaccharide wiederum sind über eine beta-1,4-glykosidische Bindung zu einer polymeren Kette miteinander verbunden. Im Allgemeinen besteht ein Hyaluronsäure-Polymer aus 250 bis 50.000 Disaccharideinheiten.

Die Anmelderin hat nämlich im Rahmen der vorliegenden Erfindung überraschenderweise herausgefunden, dass sich durch die synergistische Kombination von mindestens einem virustatischen oder antiviral wirksamen Polysaccharid einerseits sowie mindestens einem vorzugsweise sauren Glykosaminoglykan, insbesondere Hyaluronsäure oder deren Salzen, andererseits in effektiver Weise die bei Heiserkeit bzw. entzündlichen Erkrankungen des Mund- und Rachenraums (z. B. Halsschmerzen) auftretenden Beschwerden, insbesondere die damit einhergehende Schmerz- und Entzündungssymptomatik sowie Mundtrockenheit und eine beeinträchtigte Phonation, lindern lassen.

Die vorliegende Erfindung weist zahlreiche weitere Vorteile und Besonderheiten auf, welche sie gegenüber dem Stand der Technik auszeichnen und nachfolgend ausführlich geschildert sind:
Die im Rahmen der vorliegenden Erfindung eingesetzten Polysaccharide zeichnen sich durch eine gute Adsorption bzw. Anheftung an die Mundschleimhaut aus, was die Ausbildung eines schützenden Films ermöglicht. Ein derartiger Film verhindert einerseits das Eindringen von Krankheitserregern, wie beispielsweise Viren und Bakterien, und beugt andererseits durch seine stark wasserbindenden Eigenschaften Mundtrockenheit bzw. einem Austrocknen der Schleimhäute im Mund- und Rachenraum vor. Weiterhin zeichnen sich die eingesetzten Polysaccharide durch ihre hervorragende Verträglichkeit aus, da sie üblicherweise zumindest im Wesentlichen nebenwirkungsfrei sind.

Wie zuvor bereits erwähnt, sind vorzugsweise saure Glykosaminoglykane, insbesondere Hyaluronsäure und deren Salze, ebenfalls in der Lage, große Mengen Wasser zu binden und sozusagen eine Matrix auf Basis eines Gels auszubilden. Auf diese Art und Weise wird die Benetzung der Schleimhaut bzw. die Ausbildung eines schützenden Films auf der Schleimhaut, wie er bereits durch die eingesetzten Polysaccharide entsteht, noch weitergehend verstärkt. Insbesondere ergänzen sich die Polysaccharide einerseits sowie die Hyaluronsäure andererseits in synergistischer Weise.

Darüber hinaus zeichnet sich die erfindungsgemäße Zusammensetzung gegenüber den aus dem Stand der Technik bekannten Methoden zur Linderung von Heiserkeit oder Halsschmerzen bzw. entzündlichen Erkrankungen im Mund- und Rachenraum durch eine schnell eintretende und dennoch äußerst lang anhaltende Wirkung aus. Auch einige Stunden nach Applikation der Zusammensetzung besteht weiterhin eine gute Benetzung der Schleimhäute und darüber hinaus eine für den Patienten angenehme Linderung der Schmerzsymptomatik.

Zudem bewirkt die erfindungsgemäße Zusammensetzung insgesamt eine äußerst schnelle Regeneration bzw. Wiederherstellung der Phonation, d. h. die stimmlichen Beeinträchtigungen, insbesondere die belegte, raue oder heisere Stimme, werden schnell geheilt. Auch andere entzündliche Erkrankungen des Mund- und Rachenraums (z. B. Halsschmerzen) lassen sich mit der erfindungsgemäßen Zusammensetzung in effizienter Weise behandeln.

Darüber hinaus zeichnet sich die erfindungsgemäße Zusammensetzung durch eine hervorragende Verträglichkeit aus, da sie zumindest im Wesentlichen frei von etwaigen Nebenwirkungen ist. Aufgrund der guten Verträglichkeit ist die erfindungsgemäße Zusammensetzung somit auch für einen verhältnismäßig lang andauernden Gebrauch bzw. Einsatz sowie zur Verwendung bei Kindern geeignet.

Schließlich wird im Zusammenhang mit den zuvor beschriebenen Vorteilen und Besonderheiten bereits an dieser Stelle auf die von der Anmelderin durchgeführten Wirksamkeitsstudien verwiesen, welche die vorgenannten Effekte in eindrucksvoller Weise belegen und nachfolgend noch detailliert beschrieben sind.

Die erfindungsgemäße Zusammensetzung kann in vielfältiger Art und Weise ausgestaltet sein. Bevorzugte Ausführungsformen sind im Folgenden zum besseren Verständnis beschrieben.

Um eine gute Anheftung des Polysaccharids an die Mundschleimhaut bzw. Rachenschleimhaut zu erzielen, hat es sich als vorteilhaft erwiesen, Polysaccharide mit definierten gewichtsmittleren Molekulargewichten einzusetzen. Gemäß einer bevorzugten Ausführungsform der vorliegenden Erfindung kann es somit vorgesehen sein, dass die Komponente (a) ein gewichtsmittleres Molekulargewicht im Bereich von 5.000 bis 10.000.000 Da, insbesondere im Bereich von 15.000 bis 7.500.000 Da, vorzugsweise im Bereich von 20.000 bis 5.000.000 Da, bevorzugt im Bereich von 30.000 bis 3.000.000 Da, aufweist. Die Bestimmung des Molekulargewichts erfolgt vorzugsweise mittels Gelpermeationschromatographie (GPC), vorzugsweise nach DIN 55672.

Um darüber hinaus eine besonders gute Verträglichkeit sicherzustellen, hat es sich insbesondere als vorteilhaft erwiesen, wenn die Komponente (a) ein Biopolymer bzw. ein Polymer biogenen Ursprungs ist.

Wie bereits zuvor erwähnt, können Polysaccharide sowohl als Homopolysaccharide bzw. Homoglykane als auch als Heteropolysaccharide bzw. Heteroglykane vorliegen. Erfindungsgemäß kann es somit vorgesehen sein, dass die Komponente (a) aus Homopolysacchariden und/oder Heteropolysacchariden ausgewählt ist. Üblicherweise handelt es sich bei Polysacchariden mit gelbildenden Eigenschaften um Heteropolysaccharide, was sich vor dem Hintergrund einer guten Adsorption bzw. Anheftung an die Mundschleimhaut im Rahmen der vorliegenden Erfindung als vorteilhaft erwiesen hat. Bevorzugt ist somit im Rahmen der vorliegenden Erfindung der Einsatz von Heteropolysacchariden.

Eine besonders gute Adsorption an die Mundschleimhaut und somit ein hervorragender Schutz der Schleimhaut vor Austrocknung einerseits sowie eindringenden Keimen andererseits kann erzielt werden, wenn die Komponente (a) aus Schleimstoffen, insbesondere Galactansulfaten und/oder deren Derivaten und/oder deren physiologisch verträglichen Salzen und/oder Polyuroniden und/oder deren Derivaten und/oder deren physiologisch verträglichen Salzen ausgewählt ist.

Gemäß einer bevorzugten Ausführungsform der vorliegenden Erfindung kann es in diesem Zusammenhang vorgesehen sein, dass die Komponente (a), insbesondere das Polysaccharid, vorzugsweise als Schleimstoff, insbesondere in Form einer Droge, insbesondere in Form zerkleinerter oder pulverisierter oder extrahierter Algen, Pflanzenteile oder Pflanzenbestandteile, bereitgestellt ist.

Gemäß einer weiteren bevorzugten Ausführungsform der erfindungsgemäßen Zusammensetzung kann es vorgesehen sein, dass die Komponente (a), insbesondere das Polysaccharid, vorzugsweise als Schleimstoff, in Form eines Extraktes, insbesondere eines Trockenextraktes, bereitgestellt ist. Vorzugsweise wird der Extrakt ausgehend von einem wässrigen, alkoholischen oder wässrig-alkoholischen Extrakt, bevorzugt einem wässrigen Extrakt, erhalten.

In diesem Zusammenhang lässt sich die Wirksamkeit der erfindungsgemäßen Zusammensetzung noch weitergehend steigern, wenn der Extrakt, vorzugsweise der Trockenextrakt, ein Droge/Extrakt-Verhältnis (DEV), insbesondere berechnet als gewichtsbezogenes Verhältnis von eingesetzter Ausgangsmenge an Droge zu erhaltenem Extrakt, im Bereich von 0,1 : 1 bis 50: 1, insbesondere im Bereich von 0,5 : 1 bis 25 : 1, vorzugsweise im Bereich von 2 : 1 bis 20 : 1, besonders bevorzugt im Bereich von 5 : 1 bis 10 : 1, aufweist.

Das sogenannte Droge/Extrakt-Verhältnis (DEV) charakterisiert das (gewichtsbezogene) Verhältnis von eingesetzter Ausgangsdroge zu erhaltenem Extrakt. Das Droge/Extrakt-Verhältnis (DEV) gibt also an, aus welcher gewichtsbezogenen Menge an eingesetzter Droge (z. B. Rotalgen) welche gewichtsbezogene Menge an Extrakt gewonnen wurde. Ein Droge/Extrakt-Verhältnis beispielsweise von 10 : 1 bedeutet, dass aus 10 Gewichtsteilen Droge ein Gewichtsteil Extrakt gewonnen wurde. Das Droge/Extrakt-Verhältnis gibt also an, wieviele Gewichtsteile einer Arzneidroge für die Herstellung des gewichtsbezogenen Extraktäquivalents benötigt werden.

Die Verwendung von Extrakten mit definiertem Droge/Extrakt-Verhältnis ist insbesondere vor dem Hintergrund relevant, dass die Qualität des Extraktes einen entscheidenden Einfluss auf die Gesamtqualität der pharmazeutischen Zusammensetzung hat. Ziel ist es dabei zudem, den Extrakt hinsichtlich der Wirkstoffkonzentration zu standardisieren, so dass auch die finale pharmazeutische Zubereitung qualitativ und quantitativ mit konstant guten Werten bezüglich der Wirk- und Inhaltsstoffe bereitgestellt werden kann.

Was die Komponente (a) bzw. das Polysaccharid bzw. den Schleimstoff weiterhin anbelangt, so ist es im Rahmen der vorliegenden Erfindung bevorzugt, wenn dieser erhältlich ist aus Isländischem Moos (*Lichen islandicus*), Eibisch (*Althaea officinalis L.*), Spitzwegerich (*Plantago lanceolata L*.), Malve (*Malva sylvestris L.* und *M. neglecta WALLR.*), Boxhornklee (*Trigonella foenum-graecum L*.), Salep, Quitte (*Cydonia oblonga MILL.*) und Algen, vorzugsweise Rotalgen, insbesondere Rotalgen-Spezies der Gattungen *Chondrus, Euchema* und/oder *Gigartina,* sowie deren Kombinationen.

Isländisches Moos ist der deutsche Name für *Lichen islandicus* oder *Cetraria islandica* (*Parmeliaceae*), einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindernde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, bei Appetitlosigkeit sowie bei Schleimhautreizungen im Mund- und Rachenraum angewandt. Ein Mund- und Rachendesinfiziens auf Basis von Isländischem Moos wirkt reizlindernd bei trockenem Reizhusten. Das Kaltmazerat und andere bitterschmeckende Zubereitungen der Droge werden zur Behebung der Sub- oder Antazidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Lichen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Der Eibisch (*Althaea officinalis L*.), eingesetzt in Form der Eibischwurzel, -blätter und -blüten, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälte Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wässrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirupus Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Der Spitzwegerich (*Plantago lanceolata L*.) ist in ganz Europa sowie in Nord- und Mittelasien verbreitet. Die Droge stammt von wild wachsenden Pflanzen und Kulturen vor allem Südosteuropas. Die getrockneten, oliv- bis bräunlichgrün gefärbten, lanzettlichen Blattspreiten mit etwa drei bis sieben parallel verlaufenden Nerven können verwendet werden. Die Herba-Droge besteht aus den getrockneten Blättern, Stengeln und ganzen Blüten. Neben dem Schleim enthält der Spitzwegerich als weitere Inhaltsstoffe Tannine, das Iridoidglykosid Aucubin (1,9 bis 2,4 %), das für die Dunkelfärbung einer nicht sorgfältig getrockneten Droge verantwortlich ist und zur Identitätsprüfung herangezogen wird, ferner das Senföl Sulforaphen. Der Spitzwegerich, insbesondere das Spitzwegerichblätter-Kraut, findet insbesondere Anwendung in Form von Tees und Sirupen bei Entzündungen des Mund- und Rachenraums.

Die Malve (*Malva sylvestris L.* und *M. neglecta WALLR.*), insbesondere eingesetzt in Form von Malvenblüten und -blättern, ist in Europa, Kleinasien, im Mittelmeergebiet und in Vorderindien heimisch. Zur Anwendung kommen die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expectorans verwendet.

Der Bockshornklee (*Trigonella foenum-graecum L*.), insbesondere eingesetzt in Form des Bockshornkleesamens, ist im gesamten Mittelmeergebiet, sowie in Osteuropa, Indien und China beheimatet. Der Hauptdrogenimport stammt aus den Kulturen Indiens und Marokkos. Zur Anwendung kommen die braun-rötlich gefärbten, vierseitigen und rautenförmigen, etwa 5 mm langen und etwa 3 mm breiten, flachgedrückten, sehr harten Samen, die durch eine Furche charakterisiert sind. Die Droge enthält außer fettem Öl 20 bis 30 % Schleim, Trigonellin, Nicotinsäureamid, Cholin, Bitterstoffe und Saponine. Die Herstellung des Schleims erfolgt aus dem gepulvertem Samen. Das Pulver wird angewendet äußerlich zu Umschlägen bei Furunkeln, Geschwüren und Drüsenschwellungen, innerlich als Expectorans und Roborans.

Salep bzw. Salep Tuber, insbesondere eingesetzt in Form der Salepknolle, ist von verschiedenen Orchideen-Arten abgeleitet, besonders *Orchis mascula L., Orchis morio L., Orchis militaris L., Anacamptis pyramidalis L. RICH.* und *Platanthera bifolia L. RICH.* Zur Anwendung kommen die etwa 4 cm langen bis etwa 3 cm dicken, runzeligen, harten, bräunlichen Knollen, die nach der Ernte von der Korbschicht befreit, mit siedendem Wasser abgebrüht und bei künstlicher Wärme getrocknet werden. Salepschleim, der bis zu 50 % vorwiegend aus Glucomannan bzw. Glucan besteht, wird hauptsächlich in der Kinderheilpraxis als Antidiarrhoikum verwendet.

Die Quitte (*Cydonia oblonga MILL*.) ist im südöstlichen Arabien heimisch. Hautdrogenimporte kommen aus Spanien, Portugal und Persien. Zur Anwendung kommen die reifen, getrockneten, rot-braun bis braun-violett gefärbten, etwas abgeplatteten Samen der Quittenscheinfrucht. Die Samen sind außen zum Teil mit einer eingetrockneten Schleimkruste versehen und oft miteinander verklebt. Sie besitzen einen schwachen Bittermandelgeschmack. In der Epidermis findet sich ca. 22 % Schleim, der zum größten Teil wasserlöslich ist. Die Zuckerkomponenten sind Arabinose, Xylose und Uronsäure, die zum Teil methyliert ist. Die Droge kommt nur unzerkleinert zur Anwendung. Der Quittenschleim wird äußerlich bei Lippen- und Brustwarzenrhagaden, bei Verbrennungen und Dekubitus in Salben- oder Cremeform verwendet.

Unter der Bezeichnung Algen werden im weiteren Sinne im Wasser lebende, eukaryotische, pflanzenartige Lebewesen bezeichnet, welche Photosynthese betreiben, jedoch den eigentlichen Pflanzen zugerechnet werden. Algen zeichnen sich durch einen besonders vielfältigen Stoffwechsel aus, welcher unter anderem die Synthese zahlreicher Polysaccharide vorsieht. Bei den von Algen synthetisierten Polysacchariden handelt es sich insbesondere um Schleimstoffe und Verdickungsmittel, welche bislang vor allem in der Lebensmittel- sowie Kosmetikindustrie Anwendung finden. Insbesondere Rotalgen (*Rhodophyta*) zeichnen sich durch ihre Fähigkeit zur Synthese von Polymeren, wie Agar auf Basis von Agarose, sulfoniertem Agaropektin sowie Carrageen auf Basis von sulfonierter Galaktose und 3,6-Anhydrogalaktose, aus. Sowohl Agar als auch Carrageen bilden bei Kontakt mit Wasser aufgrund ihrer Fähigkeit, Wasser in großen Mengen zu binden, eine gallertartige Masse bzw. einen sogenannten Schleim.

Gemäß einer besonders bevorzugten Ausführungsform der vorliegenden Erfindung kann es vorgesehen sein, dass die Komponente (a), insbesondere das Polysaccharid, vorzugsweise der Schleimstoff, aus den Carrageenen und/oder deren Derivaten und/oder deren physiologisch verträglichen Salzen, insbesondere alpha-, iota-, gamma-, kappa- und/oder lambda-Carrageenen und/oder deren Derivaten und/oder deren physiologisch verträglichen Salzen, vorzugsweise iota-Carrageenen und/oder dessen Derivaten und/oder physiologisch verträglichen Salzen, ist.

Gleichermaßen kann es vorgesehen sein, dass die Komponente (a), insbesondere das Polysaccharid, vorzugsweise der Schleimstoff, ein Heteropolymer auf Basis von alpha-, iota-, gamma-, kappa- und/oder lambda-Carrageen und/oder deren Derivaten und/oder deren physiologisch verträglichen Salzen, insbesondere auf Basis von iota- und kappa-Carrageen und/oder deren Derivaten und/oder deren physiologisch verträglichen Salzen, gebildet ist.

Im Rahmen der vorliegenden Erfindung wird unter dem Begriff "Carrageen" bzw. "Carrageenan" eine Sammelbezeichnung für eine Gruppe langkettiger Polysaccharide verstanden, welche insbesondere in Zellen verschiedener Rotalgenarten vorkommen bzw. synthetisiert werden. Bei Carrageenen handelt es sich um lineare anionische Hydrokolloide, wobei je nach chemischer Struktur verschiedene Carrageentypen unterschieden werden. Aufgrund der jeweiligen Struktur ergeben sich für die verschiedenen Typen auch unterschiedliche Eigenschaften. Die Zuordnung erfolgt in erster Linie anhand des Anteils an Galaktose und 3,6-Anhydrogalaktose sowie der Anzahl an Sulfatgruppen. Zur Herstellung von Carrageen aus Rotalgen werden diese zunächst gewaschen und in alkalischer Lösung gekocht. Anschließend wird die Lösung filtriert, um die Zelltrümmer bzw. Algenbestandteile von dem Polymer zu separieren. Zur Gewinnung des Carrageens aus der Lösung wird das Carrageen schließlich entweder mittels Alkohol ausgefällt oder mittels Kaliumchlorid geliert und anschließend abgepresst. Das gewonnene Carrageen kann anschließend getrocknet, vermahlen und je nach Einsatzzweck weiterverarbeitet werden. Von besonderem kommerziellen Interesse sind insbesondere kappa-, iota- und lambda-Carrageen. Dennoch können alternativ auch andere Carrageentypen eingesetzt werden, welche dann insbesondere als Vorstufen für kappa-, iota- und lambda-Carrageen dienen.

Im Rahmen der vorliegenden Erfindung hat sich überraschenderweise gezeigt, dass durch den Einsatz von Carrageen als Polysaccharid einerseits hervorragende antivirale Wirkungen bei Anwendung auf der Schleimhaut im Hals- und Rachenraum erzielt werden können; andererseits bildet Carrageen aufgrund seiner hydrokolloidartigen Eigenschaften einen hervorragenden Schutzfilm auf der Mund- und Rachenschleimhaut, um Mundtrockenheit bzw. einem Austrocknen der Schleimhäute in effizienter Weise vorzubeugen und das Eindringen von Krankheitserregern verhindern.

Im Zusammenhang mit dem mindestens einen Polysaccharid bzw. der Komponente (a) hat es sich zudem als vorteilhaft erwiesen, die Substanz in definierten Mengen einzusetzen. Als besonders vorteilhaft in Bezug auf die Wirksamkeit hat es sich erwiesen, wenn die Zusammensetzung die Komponente (a) in einer relativen Menge im Bereich von 0,1 bis 40 Gew.-%, insbesondere im Bereich von 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung, enthält.

Diesbezüglich hat es sich als vorteilhaft erwiesen, wenn die Zusammensetzung die Komponente (a) in einer absoluten Menge im Bereich von 0,001 bis 5 g, insbesondere im Bereich von 0,005 bis 4 g, vorzugsweise im Bereich von 0,01 bis 3 g, bevorzugt im Bereich von 0,05 bis 2 g, insbesondere bezogen auf eine Applikationseinheit (z. B. Lutschtablette) und/oder insbesondere auf eine Applikationsmenge, enthält.

Was konkret die Komponente (b) bzw. das mindestens eine vorzugsweise saure Glykosaminoglykan anbelangt, so hat es sich im Rahmen der vorliegenden Erfindung als besonders vorteilhaft erwiesen, wenn die Zusammensetzung Hyaluronsäure oder deren physiologisch unbedenkliche Salze, vorzugsweise das Natriumsalz der Hyaluronsäure, enthält.

Die eingesetzte Menge der Komponente (b) kann erfindungsgemäß in weiten Bereichen variieren. Besonders gute Ergebnisse werden erzielt, wenn die Zusammensetzung die Komponente (b) in einer Menge im Bereich von 0,001 bis 20 Gew.-%, insbesondere im Bereich von 0,005 bis 10 Gew.-%, vorzugsweise im Bereich von 0,01 bis 5 Gew.-%, bevorzugt im Bereich von 0,1 bis 2 Gew.-%, besonders bevorzugt im Bereich von 0,2 bis 1 Gew.-%, bezogen auf die Zusammensetzung, enthält.

Gleichermaßen kann es vorgesehen sein, dass die Zusammensetzung die Komponente (b) in einer absoluten Menge im Bereich von 0,1 bis 1.000 mg, insbesondere im Bereich von 0,5 bis 500 mg, vorzugsweise im Bereich von 0,1 bis 100 mg, bevorzugt im Bereich von 1 bis 10 mg, insbesondere bezogen auf eine Applikationseinheit (z. B. Lutschtablette) und/oder insbesondere bezogen auf eine Applikationsmenge, enthält.

Darüber hinaus hat es sich im Rahmen der vorliegenden Erfindung als vorteilhaft in Bezug auf die Wirksamkeit der erfindungsgemäßen Kombination erwiesen, wenn das Polysaccharid (a) einerseits sowie das vorzugsweise saure Glykosaminoglykan (b) andererseits in definierten gewichtsbezogenen Verhältnissen zueinander in der Zusammensetzung vorliegen. Erfindungsgemäß ist es bevorzugt, wenn die Zusammensetzung die Komponente (a) und die Komponente (b) in einem gewichtsbezogenen Verhältnis von [(a) : (b)] im Bereich von 1 : 1 bis 1.000 : 1, insbesondere im Bereich von 2 : 1 bis 500 : 1, vorzugsweise im Bereich von 5 : 1 bis 100 : 1, bevorzugt im Bereich von 10 : 1 bis 75 : 1, enthält.

Überraschenderweise wurde im Rahmen der vorliegenden Erfindung zudem herausgefunden, dass die Wirksamkeit der erfindungsgemäßen Zusammensetzung noch weitergehend gesteigert werden kann, wenn diese als weitere Komponente (c) mindestens einen Schaumbildner enthält. Ein Schaumbildner führt durch seine oberflächenaktiven Eigenschaften zu einer besonders gleichmäßigen bzw. flächendeckenden und starken Benetzung der Schleimhaut, was darüber hinaus mit einer Wirkungsverstärkung des Polysaccharids sowie des sauren Glykosaminoglykans verbunden ist. Darüber hinaus erzeugt der Schaumbildner ein angenehmes Mundgefühl. Vorzugsweise ist die Komponente (c) bzw. der Schaumbildner auf Basis mindestens eines mehrwertigen Alkohols oder dessen physiologisch unbedenklichen Derivaten oder Estern, insbesondere eines dreiwertigen Alkohols oder dessen physiologisch unbedenklichen Derivaten oder Estern, vorzugsweise Glycerin oder dessen physiologisch unbedenklichen Derivaten oder Estern, ausgebildet.

Was die Mengen des Schaumbildners bzw. der Komponente (c) in der erfindungsgemäßen Zusammensetzung anbelangt, so können diese gleichermaßen in weiten Bereichen variieren. Vorzugsweise enthält die Zusammensetzung die Komponente (c) in einer relativen Menge im Bereich von 0,001 bis 10 Gew.-%, insbesondere im Bereich von 0,005 bis 5 Gew.-%, vorzugsweise im Bereich von 0,01 bis 2,5 Gew.-%, bevorzugt im Bereich von 0,1 bis 1 Gew.-%, bezogen auf die Zusammensetzung.

In Bezug auf den Schaumbildner hat es sich als vorteilhaft erwiesen, wenn dieser in definierten gewichtsbezogenen Verhältnissen zu der Komponente (a) bzw. dem Polysaccharid sowie der Komponente (b) bzw. dem mindestens einen vorzugsweise sauren Glykosaminoglykan eingesetzt wird. Besonders gute Ergebnisse werden erhalten, wenn die Zusammensetzung die Komponente (a) und die Komponente (c) in einem gewichtsbezogenen Verhältnis von [(a): (c)] im Bereich von 1 : 1 bis 1.000 : 1, insbesondere im Bereich von 2 : 1 bis 500 : 1, vorzugsweise im Bereich von 5 : 1 bis 100 : 1, bevorzugt im Bereich von 10: 1 bis 75 : 1 enthält. Weiterhin kann es vorgesehen sein, dass die Zusammensetzung die Komponente (b) und die Komponente (c) in einem gewichtsbezogenen Verhältnis von [(b): (c)] im Bereich von 1 : 100 bis 100 : 1, insbesondere im Bereich von 1 : 50 bis 50 : 1, vorzugsweise im Bereich von 1 : 20 bis 20 : 1, bevorzugt im Bereich von 1 : 10 bis 10 : 1, besonders bevorzugt im Bereich von 1 : 5 bis 5 : 1, ganz besonders bevorzugt im Bereich von 1 : 2 bis 2 : 1, enthält.

Darüber hinaus kann die Zusammensetzung gemäß der vorliegenden Erfindung mindestens einen weiteren Wirk- und/oder Inhaltsstoff enthalten, welcher insbesondere ausgewählt sein kann aus der Gruppe von Schleimhautschutzmitteln, Antiseptika, Vitaminen, Spurenelementen, Mineralien, Mikronährstoffen sowie deren Mischungen.

Auch kann es im Rahmen der vorliegenden Erfindung vorgesehen sein, dass die Zusammensetzung außerdem mindestens ein Additiv enthalten kann; dieses Additiv kann insbesondere ausgewählt sein aus der Gruppe von Verarbeitungshilfsmitteln, Färbe-, Puffer-, Riech-, Duft-, Streck-, Binde-, Netz- und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und/oder Antiseptika sowie deren Mischungen.

Was die Darreichungsform bzw. Applikationsform der vorliegenden Erfindung anbelangt, so ist diese äußerst vielfältig und kann gezielt auf den jeweiligen Verwendungszweck maßgeschneidert bzw. angepasst werden.

Erfindungsgemäß ist es vorgesehen, dass die Zusammensetzung als feste Darreichungsform vorliegt bzw. dass die Zusammensetzung als Tablette, Dragee, Pille, Hartkaramelle oder dergleichen, insbesondere als Lutschtablette, vorliegt. In diesem Zusammenhang ist es besonders bevorzugt, wenn die Lutschtablette ein Gesamtgewicht im Bereich von 0,5 bis 5 g, insbesondere im Bereich von 0,8 bis 4 g, vorzugsweise im Bereich von 1 bis 3 g, aufweist.

Um eine gute Freisetzung der Wirk- bzw. Inhaltsstoffe zu ermöglichen, ist es bevorzugt, wenn die Zusammensetzung diese eingebettet in eine feste Matrix bzw. Masse, insbesondere eine Masse bzw. Matrix auf Basis von Zucken und/oder Zuckeraustauschstoffen, enthält. In diesem Zusammenhang kann der Zucker aus der Gruppe von Saccharose, Glukose, insbesondere Dextrose, und Fructose ausgewählt sein. Gleichermaßen kann es vorgesehen sein, dass die Zuckeraustauschstoffe aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose, besonders bevorzugt Isomalt, ausgewählt sind.

Was die eingesetzten Mengen an Zucker bzw. Zuckeraustauschstoffen anbelangt, so sind diese variabel. Im Rahmen der vorliegenden Erfindung ist es bevorzugt, wenn die eingesetzte Menge 30 bis 99,9 Gew.-%, insbesondere im Bereich von 40 bis 99 Gew.-%, vorzugsweise im Bereich von 50 bis 98 Gew. %, bezogen auf die Zusammensetzung, beträgt.

Was die feste Dosierungsform hinsichtlich ihrer physikalischen Ausgestaltung anbelangt, so ist es üblicherweise vorgesehen, dass die feste Dosierungsform eine therapeutisch wirksame Menge der Wirk- bzw. Inhaltsstoffe beim Lutschen freisetzt, wenn die feste Dosierungsform im Mund- und Rachenraum eines Patienten verabreicht und gelutscht wird.

Gegenstand der vorliegenden Erfindung - gemäß einer besonderen Ausführungsform - ist somit eine Zusammensetzung, insbesondere pharmazeutische Zusammensetzung, wie sie zuvor beschrieben wurde, in Form einer festen Dosierung, insbesondere zur topischen Behandlung von Heiserkeit und/oder entzündlichen Erkrankungen des Mund- und Rachenraums, wobei die Zusammensetzung in Kombination und jeweils in wirksamen, insbesondere pharmazeutisch wirksamen Mengen
(a) mindestens ein virustatisches oder antiviral wirksames Polysaccharid in einer relativen Menge im Bereich von 0,1 bis 40 Gew.-%, insbesondere im Bereich von 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung;
(b) Hyaluronsäure oder deren Salze in einer relativen Menge im Bereich von 0,001 bis 20 Gew.-%, insbesondere im Bereich von 0,005 bis 10 Gew.-%, vorzugsweise im Bereich von 0,01 bis 5 Gew.-%, bevorzugt im Bereich von 0,1 bis 2 Gew.-%, besonders bevorzugt im Bereich von 0,2 bis 1 Gew.-%, bezogen auf die Zusammensetzung; und
(c) mindestens einen Schaumbildner in einer relativen Menge im Bereich von 0,001 bis 10 Gew.-%, insbesondere im Bereich von 0,005 bis 5 Gew.-%, vorzugsweise im Bereich von 0,01 bis 2,5 Gew.-%, bevorzugt im Bereich von 0,1 bis 1 Gew.-%, bezogen auf die Zusammensetzung;
aufweist.

Die erfindungsgemäße Zusammensetzung, wie sie zuvor definiert wurde, eignet sich somit zu Verwendung bei der prophylaktischen bzw. therapeutischen Behandlung von Heiserkeit bzw. entzündlichen Erkrankungen des Mund- und Rachenraums, insbesondere zur Behandlung von Heiserkeit oder Halsschmerzen.

Darüber hinaus ist die pharmazeutische Zusammensetzung, wie sie zuvor definiert wurde, zur Herstellung eines Medikaments oder Therapeutikums zur phrophylaktischen bzw. therapeutischen Behandlung von Heiserkeit bzw. entzündlichen Erkrankungen des Mund- und Rachenraums, insbesondere zur topischen Behandlung von Heiserkeit oder Halsschmerzen, geeignet.

Für weitergehende Einzelheiten zu der erfindungsgemäßen Verwendung kann - zur Vermeidung unnötiger Wiederholungen - auf obige Ausführungen zu der erfindungsgemäßen Zusammensetzung verwiesen werden, welche im Bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der folgenden Ausführungsbeispiele veranschaulicht, welche die vorliegende Erfindung jedoch keinesfalls beschränken.

### Ausführungsbeispiele:

### A) Herstellung erfindungsgemäßer Zusammensetzungen in Form von Lutschtabletten auf Hartkaramellenbasis und von Vergleichszusammensetzungen gleichermaßen in Form von Lutschtabletten auf Hartkaramellenbasis

Es wurden verschiedene erfindungsgemäße und nichterfindungsgemäße Zusammensetzungen in Form von Lutschtabletten auf Hartkaramellenbasis hergestellt, wobei als Zuckeraustauschstoff zur Einlagerung bzw. Einbettung der Wirk- und Inhaltsstoffe eine Kombination von Xylitol und Mannitol eingesetzt wurde. Gleichermaßen können alternativ aber auch Fructose- und/oder Glucosesirup und/oder Süßstoffe, wie beispielsweise Acesulfam, Aspartam, Cyclamat, Saccharin und dergleichen, als Matrixmaterial verwendet werden. Die Herstellung der Lutschtabletten bzw. Hartkaramellen erfolgte in dem Fachmann an sich bekannter Weise.

Die Ausgangsstoffe (Wirkstoffe, Geschmacksstoffe, Zusatz- bzw. Hilfsstoffe etc.) wurden hierzu entsprechend der jeweiligen Rezeptur eingewogen und einzeln in das Matrixmaterial (Xylitol und Mannitol) eingemischt. Die Zugabe von Wasser war nicht notwendig. Der Vorgang fand unter Erwärmen statt. Die homogene Mischung aller Rohstoffe wurde in eine Dosiermaschine oder Dosiereinheit überführt. Dort wurden die Lutschtabletten in ihrer entsprechenden Größe hergestellt und während des Prozesses gewogen. Es resultierten Trockengewichte von 2,5 g pro Applikationseinheit bzw. pro Lutschtablette. Auf diese Weise wurden sowohl erfindungsgemäße Hartkaramellen als auch nichterfindungsgemäße Hartkaramellen zu Vergleichszwecken hergestellt.

Die erfindungsgemäßen Hartkaramellen A enthielten als Wirkstoffe 10 Gew.-% Carrageen sowie 0,2 Gew.-% Natriumhyaluronat. Die erfindungsgemäßen Hartkaramellen B enthielten als Wirkstoffe 10 Gew.-% Carrageenan, 0,2 Gew.-% Natrium-Hyaluronat sowie 0,25 Gew.-% Glycerin als Schaumbildner. Die diesbezüglichen prozentualen Gewichtsangaben beziehen sich jeweils auf das Trockengewicht der Zusammensetzung. Die vollständigen Rezepturen der erfindungsgemäßen Hartkaramellen A und B sind der nachstehenden Tabelle 1 zu entnehmen.

**Tabelle 1: Rezepturen der erfindungsgemäßen Hartkaramellen A und B**

| **Rohstoffe** | **Menge (trocken) pro Lutschtablette A in Gew.-%** | **Menge (trocken) pro Lutschtablette B in Gew.-%** |
|---|---|---|
| Glycerin | - | 0,25 |
| Carrageen | 10 | 10 |
| Na-Hyaluronat | 0,2 | 0,2 |
| Matrixbildner | 77,71 | 77,46 |
| Hilfsstoffe | 11,75 | 11,75 |
| Geschmacksbildner | 0,34 | 0,34 |
| gesamt | 100 | 100 |

Die nichterfindungsgemäßen Vergleichstabletten C und D enthielten jeweils lediglich einen einzelnen Wirkstoff: Die Vergleichstabletten C enthielten als Wirkstoff 10 Gew.-% Carrageen, bezogen auf das Trockengewicht der Zusammensetzung. Die Vergleichstabletten D hingegen enthielten lediglich 0,20 Gew.-% Natrium-Hyaluronat, bezogen auf das Trockengewicht der Zusammensetzung. Die übrigen Komponenten entsprachen jeweils der in Zusammensetzung bzw. Lutschtablette A enthaltenen Inhaltsstoffe. Der jeweils fehlende Gewichtsanteil des für Lutschtablette A zusätzlich eingesetzten Wirkstoffs wurde durch eine entsprechende Menge an Matrixbildner ersetzt.

### B) Wirksamkeits- bzw. Anwendungsstudien mit den erfndungsgemäßen Lutschtabletten und den Vergleichslutschtabletten sowie mit den herkömmlichen Methoden des Standes der Technik

Für die Anwendungs- und Wirksamkeitsstudien wurden einerseits die erfindungsgemäßen Lutschtabletten A und B sowie die nichterfindungsgemäßen Vergleichslutschtabletten C und D eingesetzt. Darüber hinaus wurden zum Vergleich Methoden des Standes der Technik zur Behandlung von unter Heiserkeit leidenden Patienten eingesetzt. Zum einen wurde einer ersten Probandengruppe dreimal täglich eine Gurgellösung auf Basis von Salzwasser und Salbei appliziert (Vergleich E). Zum anderen wurde die Wirksamkeit der erfindungsgemäßen Lutschtabletten bzw. Zusammensetzung mit der von herkömmlichen Hustenbonbons verglichen (Vergleich F).

Zur Untersuchung der Wirksamkeit der vorgenannten Zusammensetzungen bzw. Therapiemaßnahmen wurde eine Probandengruppe mit insgesamt 90 Probanden im Alter von 20 bis 70 Jahren, von denen 49 weiblich und 41 männlich waren, herangezogen. Die Probanden litten an Heiserkeit und entzündlichen Erkrankungen im Hals- und Rachenraum infolge von grippalen Infekten sowie der damit einhergehenden Schmerz- und Entzündungssymptomatik.

Jeweils 15 Probanden erhielten dreimal täglich über einen Zeitraum von 3 Tagen jeweils eine der Lutschtabletten A, B, C bzw. D. 15 weitere Probanden wendeten dreimal täglich eine Gurgellösung auf Basis von Salzwasser und Salbei an (Probandengruppe E). Weitere 15 Probanden (Probandengruppe F) erhielten dreimal täglich über einen Zeitraum von 3 Tagen handelsübliche Hals- bzw. Hustenbonbons. Im Rahmen der Behandlung wurde die jeweils getestete Zusammensetzung bzw. Therapiemaßnahme in Bezug auf den Rückgang der Beeinträchtigungen der Phonation, die Linderung der Schmerzsymptomatik, die Nachhaltigkeit der Behandlung in Bezug auf die Schmerzsymptomatik sowie die Linderung der Entzündungssymptomatik und darüber hinaus das Mundgefühl nach dem Schulnotensystem (1 = sehr gut bis 6 = ungenügend) bewertet. Die diesbezüglichen Ergebnisse sind der nachstehenden Tabelle 2 zu entnehmen.

**Tabelle 2: Bewertung der erfindungsgemäßen Zusammensetzungen sowie der Vergleichszusammensetzungen nach dem Schulnotensystem**

| **Zusammensetzung** | **Rückgang der Beeinträchtigung der Phonation** | **Linderung der Schmerzsymp tomatik** | **Nachhaltigkeit der Behandlung (Schmerzsymptomatik)** | **Linderung der Entzündungssymptomatik** | **Mundgefühl** |
|---|---|---|---|---|---|
| A (erfindungsgemäß) | 1,3 | 1,6 | 1,7 | 1,9 | 1,8 |
| B (erfindungsgemäß) | 1,1 | 1,4 | 1,5 | 1,8 | 1,2 |
| C (Vergleich) | 2,8 | 2,9 | 2,7 | 2,5 | 2,4 |
| D (Vergleich) | 3,0 | 2,9 | 2,9 | 2,7 | 2,7 |
| E (Stand der Technik) | 4,1 | 4,5 | 4,6 | 5,0 | 4,5 |
| F (Stand der Technik) | 4,5 | 4,1 | 4,7 | 5,1 | 2,7 |

Mit beiden erfindungsgemäßen Präparaten bzw. Lutschtabletten zeigte sich ein schneller Rückgang der Beeinträchtigung der Phonation sowie darüber hinaus eine hervorragende Linderung der Schmerzsymptomatik, welche darüber hinaus von den Probanden auch als äußerst nachhaltig bzw. langanhaltend empfunden wurde. Weiterhin lässt sich mit beiden erfindungsgemäßen Zusammensetzungen eine hervorragende Linderung der Entzündungssymptomatik erzielen. Neben den medizinisch relevanten Kriterien berichteten die Patienten darüber hinaus über ein äußerst angenehmes Mundgefühl der erfindungsgemäßen Zusammensetzungen. Beide erfindungsgemäßen Zusammensetzungen wurden in Bezug auf alle untersuchten Kriterien durchweg mit "sehr gut" bewertet. Dabei zeigte sich jedoch die erfindungsgemäße Lutschtablette B, welche zusätzlich 0,25 Gew. % Glycerin enthielt, gegenüber der erfindungsgemäßen Lutschtablette A noch effizienter. Insbesondere in Bezug auf den Rückgang der Beeinträchtigung der Phonation sowie das Mundgefühl kann die Wirkung der erfindungsgemäßen Zusammensetzung durch den zusätzlichen Einsatz von Glycerin noch weitergehend verbessert werden.

Mit den Vergleichszusammensetzungen C und D hingegen, welche lediglich Carrageen bzw. Natrium-Hyaluronat als Einzelwirkstoff enthielten, konnten keine zufriedenstellenden Ergebnisse erzielt werden. Die deutliche Unterlegenheit gegenüber den erfindungsgemäßen Lutschtabletten A und B lässt vielmehr den Rückschluss zu, dass sich die erfindungsgemäß eingesetzten Inhaltsstoffe - nämlich Hyaluronsäure einerseits und Polysaccharide bzw. Carrageene andererseits - in synergistischer Weise ergänzen, da die Wirkung der jeweiligen Einzelwirkstoffe bei kombiniertem Einsatz in signifikanter Weise über die jeweilige Wirksamkeit als Monopräparat hinausgeht.

Darüber hinaus hat sich im Rahmen der erfindungsgemäß durchgeführten Anwendungs- und Wirksamkeitsstudien gezeigt, dass herkömmliche Behandlungsmethoden von Heiserkeit bzw. entzündlichen Erkrankungen des Mund- und Rachenraums mit Therapiemaßnahmen des Standes der Technik, nämlich Gurgellösungen einerseits und Hustenbonbons andererseits, nur unzureichende bzw. nicht zufriedenstellende Therapieerfolge erzielt werden können.

Insgesamt geht somit aus den vorstehenden Ausführungen hervor, dass sich mit den erfindungsgemäßen Zusammensetzungen in äußerst wirkungsvoller Weise Heiserkeit bzw. entzündliche Erkrankungen des Mund- und Rachenraums behandeln lassen. Einerseits wird äußerst schnell ein Rückgang der Beeinträchtigung der Phonation erzielt. Andererseits können in effektiver Weise die Schmerzsymptomatik - insbesondere mit äußerst großer Nachhaltigkeit - sowie die Entzündungssymptomatik verbessert werden. Darüber hinaus zeichnen sich die erfindungsgemäßen Zusammensetzungen durch ein hervorragendes Mundgefühl aus, was die Einnahme der Lutschtabletten für den Patienten noch angenehmer macht.

## Patentansprüche

1. Zusammensetzung, insbesondere pharmazeutische Zusammensetzung, in Form einer festen Dosierung, insbesondere zur topischen Behandlung von Heiserkeit und/oder entzündlichen Erkrankungen des Mund- und Rachenraums,
wobei die Zusammensetzung in Kombination und jeweils in wirksamen, insbesondere pharmazeutisch wirksamen Mengen
(a) mindestens ein virustatisches oder antiviral wirksames Polysaccharid ("Komponente (a)");
(b) Hyaluronsäure oder deren Salze ("Komponente (b)"); und
(c) mindestens einen Schaumbildner ("Komponente (c)");
aufweist.

2. Zusammensetzung nach Anspruch 1,
wobei die Komponente (a) ein gewichtsmittleres Molekulargewicht im Bereich von 5.000 bis 10.000.000 Da, insbesondere im Bereich von 15.000 bis 7.500.000 Da, vorzugsweise im Bereich von 20.000 bis 5.000.000 Da, bevorzugt im Bereich von 30.000 bis 3.000.000 Da, aufweist, insbesondere bestimmt mittels Gelpermeationschromatographie (GPC), vorzugsweise nach DIN 55672; und/oder
wobei die Komponente (a) ein Biopolymer und/oder ein Polymer biogenen Ursprungs ist; und/oder
wobei die Komponente (a) ausgewählt ist aus Homopolysacchariden und/oder Heteropolysacchariden, vorzugsweise Heteropolysacchariden.

3. Zusammensetzung nach einem der vorangehenden Ansprüche,
wobei die Komponente (a) ausgewählt ist aus Schleimstoffen, insbesondere Galactansulfaten und/oder deren Derivaten und/oder deren physiologisch verträglichen Salzen und/oder Polyuroniden und/oder deren Derivaten und/oder deren physiologisch verträglichen Salzen; und/oder
wobei die Komponente (a), insbesondere das Polysaccharid, vorzugsweise als Schleimstoff, insbesondere in Form einer Droge, insbesondere in Form zerkleinerter oder pulverisierter oder extrahierter Algen, Pflanzenteile oder Pflanzenbestandteile, bereitgestellt ist.

4. Zusammensetzung nach einem der vorangehenden Ansprüche,
wobei die Komponente (a), insbesondere das Polysaccharid, insbesondere als Schleimstoff, vorzugsweise in Form eines Extrakts, insbesondere eines Trockenextrakts, bereitgestellt ist, insbesondere wobei der Extrakt ausgehend von einem wässrigen, alkoholischen oder wässrig-alkoholischen Extrakt, bevorzugt einem wässrigen Extrakt, erhältlich ist;
insbesondere wobei der Extrakt, vorzugsweise der Trockenextrakt, ein Droge/Extrakt-Verhältnis (DEV), insbesondere berechnet als gewichtsbezogenes Verhältnis von eingesetzter Ausgangsmenge an Droge zu erhaltenem Extrakt, im Bereich von 0,1 : 1 bis 50 : 1, insbesondere im Bereich von 0,5 : 1 bis 25 : 1, vorzugsweise im Bereich von 2 : 1 bis 20 : 1, besonders bevorzugt im Bereich von 5 : 1 bis 10 : 1, aufweist.

5. Zusammensetzung nach einem der vorangehenden Ansprüche,
wobei die Komponente (a), insbesondere das Polysaccharid, vorzugsweise der Schleimstoff, erhältlich ist aus Isländischem Moos (*Lichen islandicus*), Eibisch (*Althaea officinalis L.*), Spitzwegerich (*Plantago lanceolata L.*), Malve (*Malva sylvestris L.* und *M. neglecta WALLR.*), Boxhornklee (*Trigonella foenum-graecum L*.), Salep, Quitte (*Cydonia oblonga MILL.*) und Algen, vorzugsweise Rotalgen, insbesondere Rotalgen-Spezies der Gattungen *Chondrus, Euchema* und/oder *Gigartina,* sowie deren Kombinationen; und/oder
wobei die Komponente (a), insbesondere das Polysaccharid, vorzugsweise der Schleimstoff, ausgewählt ist aus den Carrageenen und/oder deren Derivaten und/oder deren physiologisch verträglichen Salzen, insbesondere alpha-, iota-, gamma-, kappa-und/oder lambda-Carrageenen und/oder deren Derivaten und/oder deren physiologisch verträglichen Salzen, vorzugsweise iota-Carrageen und/oder dessen Derivaten und/oder physiologisch verträglichen Salzen, und/oder
wobei die Komponente (a), insbesondere das Polysaccharid, vorzugsweise der Schleimstoff, ein Heteropolymer auf Basis von alpha-, iota-, gamma-, kappa- und/oder lambda-Carrageenen und/oder deren Derivaten und/oder deren physiologisch verträglichen Salzen, insbesondere auf Basis von iota- und kappa-Carrageen und/oder deren Derivaten und/oder deren physiologisch verträglichen Salzen, ist.

6. Zusammensetzung nach einem der vorangehenden Ansprüche,
wobei die Komponente (a), insbesondere das Polysaccharid, vorzugsweise der Schleimstoff, erhältlich ist durch Extraktion von Algen, vorzugsweise Rotalgen, besonders bevorzugt Rotalgen-Spezies der Gattungen *Chondrus, Euchema* und/oder *Gigartina;* und/oder wobei die Zusammensetzung die Komponente (a) in einer relativen Menge im Bereich von 0,1 bis 40 Gew.-%, insbesondere im Bereich von 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung, enthält; und/oder
wobei die Zusammensetzung die Komponente (a) in einer absoluten Menge im Bereich von 0,001 bis 5 g, insbesondere im Bereich von 0,005 bis 4 g, vorzugsweise im Bereich von 0,01 bis 3 g, bevorzugt im Bereich von 0,05 bis 2 g, insbesondere bezogen auf eine Applikationseinheit und/oder insbesondere auf eine Applikationsmenge, enthält.

7. Zusammensetzung nach einem der vorangehenden Ansprüche,
wobei die Komponente (b) das Natriumsalz der Hyaluronsäure ist; und/oder wobei die Zusammensetzung die Komponente (b) in einer relativen Menge im Bereich von 0,001 bis 20 Gew.-%, insbesondere im Bereich von 0,005 bis 10 Gew.-%, vorzugsweise im Bereich von 0,01 bis 5 Gew.-%, bevorzugt im Bereich von 0,1 bis 2 Gew.-%, besonders bevorzugt im Bereich von 0,2 bis 1 Gew.-%, bezogen auf die Zusammensetzung, enthält; und/oder
wobei die Zusammensetzung die Komponente (b) in einer absoluten Menge im Bereich von 0,1 bis 1.000 mg, insbesondere im Bereich von 0,5 bis 500 mg, vorzugsweise im Bereich von 0,1 bis 100 mg, bevorzugt im Bereich von 1 bis 10 mg, insbesondere bezogen auf eine Applikationseinheit und/oder insbesondere auf eine Applikationsmenge, enthält.

8. Zusammensetzung nach einem der vorangehenden Ansprüche,
wobei die Zusammensetzung die Komponente (a) und die Komponente (b) in einem gewichtsbezogenen Verhältnis von [(a) : (b)] im Bereich von 1 : 1 bis 1.000 : 1, insbesondere im Bereich von 2 : 1 bis 500 : 1, vorzugsweise im Bereich von 5 : 1 bis 100 : 1, bevorzugt im Bereich von 10 : 1 bis 75 : 1, enthält; und/oder
wobei die Komponente (c) auf Basis mindestens eines mehrwertigen Alkohols oder dessen physiologisch unbedenklichen Derivaten oder Estern, insbesondere eines dreiwertigen Alkohols oder dessen physiologisch unbedenklichen Derivaten oder Estern, vorzugsweise Glycerin oder dessen physiologisch unbedenklichen Derivaten oder Estern, ausgebildet ist; und/oder
wobei die Zusammensetzung die Komponente (c) in einer relativen Menge im Bereich von 0,001 bis 10 Gew.-%, insbesondere im Bereich von 0,005 bis 5 Gew.-%, vorzugsweise im Bereich von 0,01 bis 2,5 Gew.-%, bevorzugt im Bereich von 0,1 bis 1 Gew.-%, bezogen auf die Zusammensetzung, enthält.

9. Zusammensetzung nach einem der vorangehenden Ansprüche,
wobei die Zusammensetzung die Komponente (a) und die Komponente (c) in einem gewichtsbezogenen Verhältnis von [(a) : (c)] im Bereich von 1 : 1 bis 1.000 : 1, insbesondere im Bereich von 2 : 1 bis 500 : 1, vorzugsweise im Bereich von 5 : 1 bis 100 : 1, bevorzugt im Bereich von 10 : 1 bis 75 : 1, enthält; und/oder
wobei die Zusammensetzung die Komponente (b) und die Komponente (c) in einem gewichtsbezogenen Verhältnis von [(b): (c)] im Bereich von 1 : 100 bis 100 : 1, insbesondere im Bereich von 1 : 50 bis 50 : 1, vorzugsweise im Bereich von 1 : 20 bis 20 : 1, bevorzugt im Bereich von 1 : 10 bis 10 : 1, besonders bevorzugt im Bereich von 1 : 5 bis 5 : 1, ganz besonders bevorzugt im Bereich von 1 : 2 bis 2 : 1, enthält.

10. Zusammensetzung nach einem der vorangehenden Ansprüche,
wobei die Zusammensetzung mindestens einen weiteren Wirk- und/oder Inhaltsstoff, insbesondere ausgewählt aus der Gruppe von Schleimhautschutzmitteln, Antiseptika, Vitaminen, Spurenelementen, Mineralien, Mikronährstoffen sowie deren Mischungen, enthält; und/oder
wobei die Zusammensetzung außerdem mindestens ein Additiv, insbesondere ausgewählt aus der Gruppe von Verarbeitungshilfsmitteln, Färbe-, Puffer-, Riech-, Duft-, Streck-, Binde-, Netz- und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und/oder Antiseptika sowie deren Mischungen, enthält.

11. Zusammensetzung nach einem der vorangehenden Ansprüche,
wobei die Zusammensetzung als feste Darreichungsform vorliegt und/oder wobei die Zusammensetzung als Tablette, Dragee, Pille, Hartkaramelle oder dergleichen, insbesondere als Lutschtablette, vorliegt, insbesondere wobei die Lutschtablette ein Gesamtgewicht im Bereich von 0,5 bis 5 g, insbesondere im Bereich von 0,8 bis 4 g, vorzugsweise im Bereich von 1 bis 3 g, aufweist, insbesondere wobei die Zusammensetzung die Wirk- und/oder Inhaltsstoffe in einer festen Matrix und/oder Masse, insbesondere in einer Matrix und/oder Masse auf Basis von Zuckern und/oder Zuckeraustauschstoffen aufweist, insbesondere wobei die Zucker ausgewählt sind aus der Gruppe von Saccharose, Glucose, insbesondere Dextrose, und Fructose und/oder insbesondere wobei die Zuckeraustauschstoffe ausgewählt sind aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose, besonders bevorzugt Isomalt.

12. Zusammensetzung nach einem der vorangehenden Ansprüche,
wobei die Zusammensetzung die Zucker und/oder die Zuckeraustauschstoffe in einer relativen Menge im Bereich von 30 bis 99,9 Gew.-%, insbesondere im Bereich von 40 bis 99 Gew.-%, vorzugsweise im Bereich von 50 bis 98 Gew.-%, bezogen auf die Zusammensetzung, enthält; und/oder
wobei die feste Dosierungsform, insbesondere die Lutschtablette, derart gebildet ist, dass die feste Dosierungsform, insbesondere die Lutschtablette, eine therapeutisch wirksame Menge der Wirk- und/oder Inhaltsstoffe beim Lutschen freisetzt, wenn die feste Dosierungsform im Mund- und Rachenraum eines Patienten verabreicht und gelutscht wird.

13. Zusammensetzung, insbesondere pharmazeutische Zusammensetzung, insbesondere nach einem der vorangehenden Ansprüche, in Form einer festen Dosierung, insbesondere zur topischen Behandlung von Heiserkeit und/oder entzündlichen Erkrankungen des Mund- und Rachenraums, wobei die Zusammensetzung in Kombination und jeweils in wirksamen, insbesondere pharmazeutisch wirksamen Mengen
(a) mindestens ein virustatisches oder antiviral wirksames Polysaccharid in einer relativen Menge im Bereich von 0,1 bis 40 Gew.-%, insbesondere im Bereich von 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung;
(b) Hyaluronsäure oder deren Salze in einer relativen Menge im Bereich von 0,001 bis 20 Gew.-%, insbesondere im Bereich von 0,005 bis 10 Gew.-%, vorzugsweise im Bereich von 0,01 bis 5 Gew.-%, bevorzugt im Bereich von 0,1 bis 2 Gew.-%, besonders bevorzugt im Bereich von 0,2 bis 1 Gew.-%, bezogen auf die Zusammensetzung; und
(c) mindestens einen Schaumbildner in einer relativen Menge im Bereich von 0,001 bis 10 Gew.-%, insbesondere im Bereich von 0,005 bis 5 Gew.-%, vorzugsweise im Bereich von 0,01 bis 2,5 Gew.-%, bevorzugt im Bereich von 0,1 bis 1 Gew.-%, bezogen auf die Zusammensetzung;
aufweist.

14. Zusammensetzung nach einem der vorangehenden Ansprüche
zur Verwendung im Bereich der Pharmazie, Medizin oder Lebensmitteltechnik und/oder zur Verwendung in der Humanmedizin oder Veterinärmedizin und/oder zur Verwendung bei der prophylaktischen und/oder therapeutischen Behandlung von Heiserkeit und/oder entzündlichen Erkrankungen des Mund- und Rachenraums, insbesondere zur Verwendung bei der topischen Behandlung von Heiserkeit oder Halsschmerzen.

## Claims

1. Composition, in particular a pharmaceutical composition, in the form of a fixed dosage, particularly for topical treatment of hoarseness and/or inflammatory diseases of the mouth and throat,
wherein the composition comprises, in combination and in each case effective, particularly pharmaceutically effective, amounts of:
(a) at least one virustatic or antiviral polysaccharide ("component (a)");
(b) hyaluronic acid or salts thereof ("component (b)"); and
(c) at least one foaming agent ("component (c)").

2. Composition according to claim 1,
wherein component (a) has an average molecular weight in the range from 5,000 to 10,000,000 Da, particularly in the range from 15,000 to 7,500,000 Da, preferably in the range from 20,000 to 5,000,000 Da, more preferably in the range from 30,000 to 3,000,000 Da, determined in particular by means of gel permeation chromatography (GPC), preferably according to DIN 55672; and/or
wherein component (a) is a biopolymer and/or a polymer of biological origin; and/or
wherein component (a) is selected from homopolysaccharides and/or heteropolysaccharides, preferably heteropolysaccharides.

3. Composition according to any one of the preceding claims,
wherein component (a) is selected from gums, in particular galactan sulphates and/or their derivatives and/or their physiologically tolerable salts and/or polyuronides and/or their derivatives and/or their physiologically tolerable salts; and/or
wherein component (a), especially the polysaccharide, is preferably provided as mucilage, in particular in the form of a drug, in particular in the form of comminuted or pulverised or extracted algae, plant parts or plant constituents.

4. Composition according to any one of the preceding claims,
wherein component (a), especially the polysaccharide, is provided in particular as mucilage, preferably in the form of an extract, in particular a dry extract, in particular wherein the extract is obtainable from an aqueous, alcoholic or aqueous-alcoholic extract, preferably an aqueous extract;
in particular wherein the extract, preferably the dry extract, has a drug/extract ratio (DER), in particular calculated as the weight-based ratio of the initial amount of drug used to the extract obtained in the range from 0.1: 1 to 50: 1, in particular in the range from 0.5: 1 to 25: 1, preferably in the range from 2: 1 to 20: 1, more preferably in the range from 5: 1 to 10: 1.

5. Composition according to any one of the preceding claims,
wherein component (a), in particular, the polysaccharide, preferably the mucilage, is obtainable from Icelandic moss *(Lichen islandicus*), marshmallow (*Althaea officinalis L.*), plantain *(Plantago lanceolata L.),* mallow (*Malva sylvestris L.* and *M*. *neglecta WALLR*.) fenugreek *(Trigonella foenum-graecum L.),* salep, quince (*Cydonia oblonga MILL*.) and algae, preferably red algae, especially red algae species of the genera *chondrus, euchema* and/or *gigartina,* and combinations thereof; and/or
wherein component (a), especially the polysaccharide, preferably the mucilage, is selected from carrageenan and/or derivatives thereof and/or their physiologically tolerable salts, in particular alpha, iota, gamma, kappa and/or lambda-carrageenan and/or derivatives thereof and/or their physiologically tolerable salts, preferably iotacarrageenan and/or derivatives thereof and/or their physiologically tolerable salts, and/or
wherein component (a), especially the polysaccharide, preferably the mucilage, is a heteropolymer based on iota, alpha, gamma, kappa and/or lambda-carrageenan and/or derivatives thereof and/or their physiologically tolerable salts, in particular on the base of iota and kappa-carrageenan and/or derivatives thereof and/or their physiologically acceptable salts.

6. Composition according to any one of the preceding claims,
wherein component (a), especially the polysaccharide, preferably the mucilage, is obtainable by extraction of algae, preferably red algae, more preferably red algal species of the genera chondrus, euchema and/or gigartina; and/or
wherein the composition contains component (a) in a relative amount in the range from 0.1 to 40 wt .-%, in particular in the range from 0.5 to 30 wt .-%, preferably in the range from 1 to 25 wt .-%, more preferably in the range from 2 to 20 wt.-%, particularly preferably in the range from 3 to 18 wt .-%, based on the composition; and/or
wherein the composition comprises component (a) in an absolute amount in the range from 0.001 to 5 g, particularly in the range from 0.005 to 4 g, preferably in the range from 0.01 to 3 g, more preferably in the range from 0.05 to 2 g, in particular in relation to an application unit, and/or in particular to an application amount.

7. Composition according to any one of the preceding claims,
wherein component (b) is the sodium salt of hyaluronic acid; and/or
wherein the composition is preferably component (b) in a relative amount in the range from 0.001 to 20 wt .-%, in particular in the range from 0.005 to 10 wt .-%, preferably in the range from 0.01 to 5 wt .-%, more preferably in the range from 0.1 to 2 wt .-%, particularly preferably in the range from 0.2 to 1 wt .-%, based on the composition; and/or
wherein the composition comprises component (b) in an absolute amount in the range from 0.1 to 1,000 mg, in particular in the range from 0.5 to 500 mg, preferably in the range from 0.1 to 100 mg, more preferably in the range from 1 to 10 mg, in particular in relation to an application unit, and/or in particular to an application amount.

8. Composition according to any one of the preceding claims,
wherein the composition contains component (a) and component (b) in a weight-based ratio of [(a): (b)] in the range from 1: 1 to 1,000: 1, in particular in the range from 2: 1 to 500: 1, preferably in the range from 5: 1 to 100: 1, more preferably in the range from 10: 1 to 75: 1; and/or
wherein component (c) is based on at least one polyhydric alcohol or its physiologically acceptable derivatives or esters, in particular of a trihydric alcohol or its physiologically tolerable derivatives or esters, preferably glycerol or its physiologically tolerable derivatives or esters; and/or
wherein the composition contains component (c) in a relative amount in the range from 0.001 to 10 wt .-%, in particular in the range from 0.005 to 5 wt .-%, preferably in the range from 0.01 to 2.5 wt .-% , more preferably in the range from 0.1 to 1 wt .-%, based on the composition.

9. Composition according to any one of the preceding claims,
wherein the composition contains component (a) and component (c) in a weight ratio of [(a): (c)] in the range from 1: 1 to 1,000: 1, in particular in the range from 2: 1 to 500: 1, preferably in the range of 5: 1 to 100: 1, more preferably in the range from 10: 1 to 75: 1; and/or
wherein the composition contains component (b) and component (c) in a weight ratio of [(b): (c)] in the range from 1: 100 to 100: 1, in particular in the range from 1: 50 to 50: 1, preferably in the range from 1: 20 to 20: 1, more preferably in the range from 1: 10 to 10: 1, particularly preferably in the range from 1: 5 to 5: 1, most preferably in the range from 1: 2 to 2: 1.

10. Composition according to any one of the preceding claims,
wherein the composition contains at least a further active ingredient and/or substance, in particular selected from the group of mucosal protective agents, antiseptics, vitamins, trace elements, minerals, micronutrients and mixtures thereof; and/or
wherein the composition further comprises at least one additive, in particular selected from the group consisting of processing aids, colorants, buffers, fragrances, perfumes, extenders, binders, wetting agents and/or preservatives, pH adjusters, pH buffering agents, thickeners, flavours, sweeteners, acidifiers, stabilisers and/or antiseptics, and mixtures thereof.

11. Composition according to any one of the preceding claims,
wherein the composition is present as a solid dosage form and/or wherein the composition is a tablet, dragee, pill, hard caramel or the like, in particular as a lozenge, in particular wherein the lozenge has a total weight in the range of 0.5 to 5 g, particularly in the range of 0.8 to 4 g, preferably in the range 1 to 3 g, in particular wherein the composition of the active substances and/or ingredients is present in a solid matrix and/or mass, in particular in a matrix and/or composition based on sugars and/or sugar substitutes, in particular where the sugars are selected from the group of sucrose, glucose, in particular dextrose, and fructose and/or in particular wherein the sugar substitutes are selected from sugar alcohols, preferably from the group of mannitol, xylitol, sorbitol, isomalt, maltitol syrup. lactitol, leucrose, fructooligosaccharides, glucans, polyglucose, particularly preferably isomalt.

12. Composition according to any one of the preceding claims,
wherein the composition contains the sugars and/or sugar substitutes in a relative amount in the range from 30 to 99.9 wt .-%, in particular in the range from 40 to 99 wt .-%, preferably in the range from 50 to 98 wt .-% , based on the composition; and/or
wherein the solid dosage form, especially a lozenge, is so formed that the solid dosage form, especially a lozenge, releases a therapeutically-effective amount of the active ingredients and/or substances on sucking when the solid dosage form is administered in the mouth and throat of a patient and is sucked.

13. Composition, in particular a pharmaceutical composition, in particular according to one of the preceding claims, in the form of a fixed dosage, in particular for the topical treatment of hoarseness and/or inflammatory diseases of the mouth and throat, wherein the composition contains, in combination and in each case effective, especially pharmaceutically effective, amounts:
(a) at least one virustatic or antiviral polysaccharide in a relative quantity ranging from 0.1 to 40 wt .-%, in particular in the range from 0.5 to 30 wt .-%, preferably in the range from 1 to 25 wt . -%, more preferably in the range from 2 to 20 wt .-%, particularly preferably in the range from 3 to 18 wt .-%, based on the composition;
(b) hyaluronic acid or salts thereof in a relative amount in the range from 0.001 to 20 wt .-%, in particular in the range from 0.005 to 10 wt .-%, preferably in the range from 0.01 to 5 wt .-%, more preferably in range from 0.1 to 2 wt .-%, particularly preferably in the range from 0.2 to 1 wt .-%, based on the composition; and
(c) at least one foaming agent preferably in a relative amount in the range from 0.001 to 10 wt .-%, in particular in the range from 0.005 to 5 wt .-%, preferably in the range from 0.01 to 2.5 wt .-%, more preferably in the range from 0.1 to 1 wt.-%, based on the composition.

14. Composition according to any one of the preceding claims
for use in the field of pharmacy, medicine or food technology and/or
for use in human medicine or veterinary medicine, and/or
for use in the prophylactic and/or therapeutic treatment of hoarseness and/or inflammatory diseases of the mouth and throat, especially for use in the topical treatment of hoarseness or sore throat.

## Revendications

1. Composition, en particulier composition pharmaceutique, sous forme d'une posologie solide, en particulier pour le traitement topique de l'enrouement et/ou des maladies inflammatoires de la cavité buccale et du pharynx,
la composition comprenant, en combinaison, et chacun en des quantités efficaces, en particulier pharmaceutiquement efficaces
(a) au moins un polysaccharide virustatique ou à effet antiviral ("composant (a)") ;
(b) de l'acide hyaluronique ou ses sels ("composant (b)") ;
et
(c) au moins un agent moussant ("composant (c)").

2. Composition selon la revendication 1,
dans laquelle le composant (a) présente une masse moléculaire moyenne en masse comprise dans la plage de 5000 à 10 000 000 Da, en particulier dans la plage de 15 000 à 7 500 000 Da, de préférence dans la plage de 20 000 à 5 000 000 Da, préférentiellement dans la plage de 30 000 à 3 000 000 Da, déterminée en particulier par chromatographie par perméation de gel (GPC), de préférence selon DIN 55672 ; et/ou
dans laquelle le composant (a) est un biopolymère et/ou un polymère d'origine biogène ; et/ou
dans laquelle le composant (a) est choisi parmi les homopolysaccharides et/ou les hétéropolysaccharides, de préférence les hétéropolysaccharides.

3. Composition selon l'une des revendications précédentes,
dans laquelle le composant (a) est choisi parmi les substances mucilagineuses, en particulier les sulfates de galactane et/ou leurs dérivés et/ou leurs sels physiologiquement compatibles, et/ou les polyuronides et/ou leurs dérivés et/ou leurs sels physiologiquement compatibles ; et/ou
dans laquelle le composant (a), en particulier le polysaccharide, est mis à disposition de préférence sous forme d'une substance mucilagineuse, en particulier sous forme d'une drogue, en particulier sous forme d'algues, de parties de plantes ou de constituants de plantes broyés ou pulvérisés ou extraits.

4. Composition selon l'une des revendications précédentes,
dans laquelle le composant (a), en particulier le polysaccharide, est mis à disposition en particulier sous forme d'une substance mucilagineuse, de préférence sous forme d'un extrait, en particulier d'un extrait sec, en particulier dans laquelle l'extrait peut être obtenu à partir d'un extrait aqueux, alcoolique ou hydro-alcoolique, de préférence d'un extrait aqueux ;
en particulier dans lequel l'extrait, de préférence l'extrait sec, présente un rapport drogue/extrait (DEV), en particulier calculé par le rapport, en poids, de la quantité initiale utilisée de drogue à celle de l'extrait obtenu, compris dans la plage de 0,1:1 à 50:1, en particulier dans la plage de 0,5:1 à 25:1, de préférence dans la plage de 2:1 à 20:1, d'une manière particulièrement préférée dans la plage de 5:1 à 10:1.

5. Composition selon l'une des revendications précédentes,
dans laquelle le composant (a), en particulier le polysaccharide, de préférence la substance mucilagineuse, peut être obtenu à partir de mousse d'Islande (*Lichen islandicus*), de guimauve officinale (*Althaea officinalis L*.), de plantain lancéolé (*Plantago lanceolata L*.), de mauve (*Malva sylvestris L.* et *M. neglecta WALLR.*), de trigonelle fénugrec (*Trigonella foenum-graecum L.*), de salep, de coing (*Cydonia oblonga MILL.*) et d'algues, de préférence d'algues rouges, en particulier des espèces d'algues rouges des genres *Chondrus, Euchema* et/ou *Gigartina,* ainsi que de leurs combinaisons ; et/ou
dans laquelle le composant (a), en particulier le polysaccharide, de préférence la substance mucilagineuse, est choisi parmi les carragènes et/ou leurs dérivés et/ou leurs sels physiologiquement compatibles, en particulier les carragènes alpha, iota, gamma, kappa et/ou lambda et leurs dérivés et/ou leurs sels physiologiquement compatibles, de préférence le carragène iota et/ou ses dérivés et/ou ses sels physiologiquement compatibles, et/ou
dans laquelle le composant (a), en particulier le polysaccharide, de préférence la substance mucilagineuse, est un hétéropolymère à base de carragènes alpha, iota, gamma, kappa et/ou lambda et/ou de leurs dérivés et/ou de leurs sels physiologiquement compatibles, en particulier à base de carragène iota et kappa et/ou de ses dérivés et/ou de ses sels physiologiquement compatibles.

6. Composition selon l'une des revendications précédentes,
dans laquelle le composant (a), en particulier le polysaccharide, de préférence la substance mucilagineuse, peut être obtenu par extraction d'algues, de préférence d'algues rouges, d'une manière particulièrement préférée d'espèces d'algues rouges des genres *Chondrus, Euchema* et/ou *Gigartina ;* et/ou
la composition contenant les composants (a) en une quantité relative comprise dans la plage de 0,1 à 40 % en poids, en particulier dans la plage de 0,5 à 30 % en poids, de préférence dans la plage de 1 à 25 % en poids, préférentiellement dans la plage de 2 à 20 % en poids, d'une manière particulièrement préférée dans la plage de 3 à 18 % en poids, par rapport à la composition ; et/ou
la composition contenant le composant (a) en une quantité absolue comprise dans la plage de 0,001 à 5 g, en particulier dans la plage de 0,005 à 4 g, de préférence dans la plage de 0,01 à 3 g, préférentiellement dans la plage de 0,05 à 2 g, en particulier par rapport à une unité d'administration et/ou en particulier à une quantité d'administration.

7. Composition selon l'une des revendications précédentes,
dans laquelle le composant (b) est le sel de sodium de l'acide hyaluronique ; et/ou
la composition contenant le composant (b) en une quantité relative comprise dans la plage de 0,001 à 20 % en poids, en particulier dans la plage de 0,005 à 10 % en poids, de préférence dans la plage de 0,01 à 5 % en poids, préférentiellement dans la plage de 0,1 à 2 % en poids, d'une manière particulièrement préférée dans la plage de 0,2 à 1 % en poids, par rapport à la composition ; et/ou
la composition contenant le composant (b) en une quantité absolue comprise dans la plage de 0,1 à 1000 mg, en particulier dans la plage de 0,5 à 500 mg, de préférence dans la plage de 0,1 à 100 mg, préférentiellement dans la plage de 1 à 10 mg, en particulier par rapport à une unité d'administration et/ou en particulier à une quantité d'administration.

8. Composition selon l'une des revendications précédentes,
la composition contenant le composant (a) et le composant (b) selon un rapport en poids [(a) : (b)] compris dans la plage de 1:1 à 1000:1, en particulier dans la plage de 2:1 à 500:1, de préférence dans la plage de 5:1 à 100:1, préférentiellement dans la plage de 10:1 à 75:1 ; et/ou
dans laquelle le composant (c) est réalisé sur la base d'au moins un polyalcool ou ses dérivés ou esters physiologiquement inoffensifs, en particulier d'un trialcool ou de ses dérivés ou esters physiologiquement inoffensifs, de préférence le glycérol ou ses dérivés ou esters physiologiquement inoffensifs ; et/ou
la composition contenant le composant (c) en une quantité relative comprise dans la plage de 0,001 à 10 % en poids, en particulier dans la plage de 0,005 à 5 % en poids, de préférence dans la plage de 0,01 à 2,5 % en poids, préférentiellement dans la plage de 0,1 à 1 % en poids, par rapport à la composition.

9. Composition selon l'une des revendications précédentes,
la composition contenant le composant (a) et le composant (c) selon un rapport en poids [(a) : (c)] compris dans la plage de 1:1 à 1000:1, en particulier dans la plage de 2:1 à 500:1, de préférence dans la plage de 5:1 à 100:1, préférentiellement dans la plage de 10:1 à 75:1 ; et/ou
la composition contenant le composant (b) et le composant (c) selon un rapport en poids [(b) : (c)] compris dans la plage de 1:100 à 100:1, en particulier dans la plage de 1:50 à 50:1, de préférence dans la plage de 1:20 à 20:1, préférentiellement dans la plage de 1:10 à 10:1, d'une manière particulièrement préférée dans la plage de 1:5 à 5:1, d'une manière tout particulièrement préférée dans la plage de 1:2 à 2:1.

10. Composition selon l'une des revendications précédentes,
la composition contenant au moins un autre principe actif et/ou constituant, en particulier choisi dans le groupe des agents protecteurs de muqueuse, des antiseptiques, des vitamines, des oligo-éléments, des minéraux, des micronutriments, ainsi que des mélanges de ceux-ci ; et/ou
la composition contenant par ailleurs au moins un additif en particulier choisi dans le groupe des auxiliaires de mise en oeuvre, des substances colorantes, des tampons, des aromatisants, des parfums, des diluants, des liants, des mouillants et/ou des conservateurs, des agents d'ajustement du pH, des substances tampons de pH, des épaississants, des arômes, des agents de sapidité, des édulcorants et substances sucrantes, des acidifiants, des stabilisants et/ou des antiseptiques, ainsi que des mélanges de ceux-ci.

11. Composition selon l'une des revendications précédentes,
la composition se présentant sous une forme posologique solide, et/ou la composition se présentant sous forme d'un comprimé, d'une dragée, d'une pilule, d'un caramel dur ou analogues, en particulier d'un comprimé à sucer, en particulier dans laquelle le comprimé à sucer présente une masse totale comprise dans la plage de 0,5 à 5 g, en particulier dans la plage de 0,8 à 4 g, de préférence dans la plage de 1 à 3 g, en particulier la composition comprenant les principes actifs et/ou constituants dans une matrice et/ou une masse solides, en particulier dans une matrice et/ou une masse à base de sucres et/ou de succédanés du sucre, en particulier dans laquelle les sucres sont choisis dans le groupe consistant en le saccharose, le glucose, en particulier le dextrose, et le fructose, et/ou en particulier dans laquelle les succédanés du sucre sont choisis parmi les alcools de sucre, de préférence dans le groupe consistant en le mannitol, le xylitol, le sorbitol, l'isomaltol, le sirop de maltitol, le lactitol, le leucrose, les fructo-oligosaccharides, les glucanes, le polyglucose, d'une manière particulièrement préférée l'isomaltol.

12. Composition selon l'une des revendications précédentes,
la composition contenant les sucres et/ou les succédanés du sucre en une quantité relative comprise dans la plage de 30 à 99,9 % en poids, en particulier dans la plage de 40 à 99 % en poids, de préférence dans la plage de 50 à 98 % en poids, par rapport à la composition ; et/ou
dans laquelle la forme posologique solide, en particulier le comprimé à sucer, est formée de telle sorte que la forme posologique solide, en particulier le comprimé à sucer, libère une quantité thérapeutiquement efficace des principes actifs et/ou des constituants lors du suçage, quand la forme posologique solide est administrée et sucée dans la cavité buccale et le pharynx.

13. Composition, en particulier composition pharmaceutique, en particulier selon l'une des revendications précédentes, sous une forme posologique solide, en particulier pour le traitement topique de l'enroulement et/ou des maladies inflammatoires de la cavité buccale et du pharynx, la composition comprenant, en combinaison et chacun en une quantité efficace, en particulier pharmaceutiquement efficace
(a) au moins un polysaccharide virustatique ou à effet antiviral, en une quantité relative comprise dans la plage de 0,1 à 40 % en poids, en particulier dans la plage de 0,5 à 30 % en poids, de préférence dans la plage de 1 à 25 % en poids, préférentiellement dans la plage de 2 à 20 % en poids, d'une manière particulièrement préférée dans la plage de 3 à 18 % en poids, par rapport à la composition ;
(b) de l'acide hyaluronique ou ses sels, en une quantité relative comprise dans la plage de 0,001 à 20 % en poids, en particulier dans la plage de 0,005 à 10 % en poids, de préférence dans la plage de 0,01 à 5 % en poids, préférentiellement dans la plage de 0,1 à 2 % en poids, d'une manière particulièrement préférée dans la plage de 0,2 à 1 % en poids, par rapport à la composition ; et/ou
(c) au moins un agent moussant en une quantité relative comprise dans la plage de 0,001 à 10 % en poids, en particulier dans la plage de 0,005 à 5 % en poids, de préférence dans la plage de 0,01 à 2,5 % en poids, préférentiellement dans la plage de 0,1 1 à 1 % en poids, par rapport à la composition.

14. Composition selon l'une des revendications précédentes
pour une utilisation dans le domaine de la pharmacie, de la médecine ou de l'industrie alimentaire, et/ou
pour une utilisation en médecine humaine et/ou en médecine vétérinaire, et/ou
pour une utilisation lors du traitement prophylactique et/ou thérapeutique de l'enrouement et/ou des maladies inflammatoires de la cavité buccale et du pharynx, en particulier pour une utilisation lors du traitement topique de l'enrouement ou des douleurs du cou.
